# EUROPÄISCHE PATENTSCHRIFT

(11) **EP 2 825 109 B1**
(45) Veröffentlichungstag und Bekanntmachung des Hinweises auf die Patenterteilung: **11.05.2016**
(21) Anmeldenummer: 13708820.9
(22) Anmeldetag: 11.03.2013
(51) Int. Cl.: A61B 17/34

(54) **INSTRUMENTENSYSTEM FÜR DIE MINIMALINVASIVE CHIRURGIE IN DER SINGLE-PORT-TECHNIK**
INSTRUMENT SYSTEM FOR MINIMALLY INVASIVE SURGERY IN SINGLE PORT TECHNOLOGY
SYSTÈME D'INSTRUMENTS POUR LA CHIRURGIE MINI-INVASIVE SELON LA TECHNIQUE SINGLE-PORT

(30) Priorität: 13.03.2012 DE 102012203908
(43) Veröffentlichungstag der Anmeldung: 21.01.2015
(73) Patentinhaber: Gaiselmann, Thomas, 78667 Villingendorf (DE); Riek, Siegfried, 78628 Rottweil (DE); Bachmann, Karl-Heinz, 78667 Villingendorf (DE)
(72) Erfinder: Gaiselmann, Thomas, 78667 Villingendorf (DE); Riek, Siegfried, 78628 Rottweil (DE); Bachmann, Karl-Heinz, 78667 Villingendorf (DE)
(74) Vertreter: Westphal, Mussgnug & Partner Patentanwälte mbB
(86) Internationale Anmeldenummer: PCT/EP2013/054914
(87) Internationale Veröffentlichungsnummer: WO 2013/135646

(56) Entgegenhaltungen:
- US-A1- 2005 234 297
- US-A1- 2006 178 560
- US-A1- 2007 293 727
- US-A1- 2011 118 545

## Beschreibung

Die Erfindung betrifft ein Instrumentensystem für die minimalinvasive Chirurgie in der Single-Port-Technik gemäß dem Oberbegriff des Patentanspruchs 1.

In der minimalinvasiven Chirurgie wird durch eine Inzision ein Zugang zu einer Körperhöhle geschaffen, z. B. bei der laparoskopischen Chirurgie in den Bauchraum. Durch solche Zugänge werden die für die Operation benötigten Instrumente und eine Optik, die zum Ausleuchten des intrakorporalen Operationsfeldes dient und in der Regel über eine Kamera ein Bild des Operationsfeldes zu einem Monitor überträgt, eingeführt. Während bisher für jedes Instrument und für die Optik jeweils ein gesonderter Zugang geschaffen wurde, wird zunehmend die Single-Port-Technik angewendet, bei welcher nur ein einziger Zugang (Single-Port) geschaffen wird, durch den die Instrumente und die Optik eingeführt werden können.

Für die Single-Port-Technik ist es bekannt (z.B. DE 10 2009 018 639 A1), einen Adapter in die Bauchdecke zu implantieren, durch welchen die Instrumente und die Optik eingeführt werden. Für das Implantieren des Adapters ist ein relativ großer Hautschnitt notwendig, was der Zielsetzung der minimalinvasiven Chirurgie entgegensteht. Die Implantation erfordert einen aufwändigen Operationsschritt, weil der Adapter luftdicht in die Bauchdecke eingesetzt und fixiert werden muss. Weiter liegen die Instrumente und die Optik im Wesentlichen achsparallel und im geringen seitlichen Abstand zueinander, sodass die Durchführung der operativen Schritte mittels der Instrumente eingeschränkt ist.

Aus der US 2011/0118545 A1 ist ein Instrumentensystem der eingangs genannten Gattung bekannt, bei welchem Instrumentenkanäle und eine Optik durch eine gemeinsame Single-Port-Hülse eingeführt werden. Durch die Instrumentenkanäle werden flexible Instrumente eingeführt, wobei deren Betätigungsgriff extrakorporal verbleibt und das distale Arbeitselement der Instrumente distal aus dem Instrumentenkanal austritt. Die Instrumentenkanäle können über ein Seilzugsystem gesteuert werden, um die Arbeitselemente der Instrumente zu positionieren. Die Steuerung der Instrumentenkanäle ist konstruktiv aufwendig, was einerseits die Reinigung und Sterilisation erschwert und andererseits einer Einmal-Verwendung aus Kostengründen entgegensteht.

Der Erfindung liegt die Aufgabe zu Grunde, ein Instrumentensystem für die minimalinvasive Chirurgie in der Single-Port-Technik zur Verfügung zu stellen, welches nur eine kleine Inzision notwenig macht, kostengünstig herstellbar ist und die Handhabung der Instrumente mit vielen Freiheitsgraden ermöglicht.

Diese Aufgabe wird erfindungsgemäß gelöst durch ein Instrumentensystem mit den Merkmalen des Patentanspruchs 1.

Vorteilhafte Ausführungen der Erfindung sind in den Unteransprüchen angegeben.

Das erfindungsgemäße Instrumentensystem weist einen Trokar auf, der zur Schaffung des Zugangs dient. Der Trokar kann einen beliebigen aus dem Stand der Technik bekannten grundsätzlichen Aufbau aufweisen. Der Begriff "Trokar" wird hier allgemein verwendet. Es kann sich um einen aus Trokardorn und Trokarhülse bestehenden Trokar handeln. Ebenso kann der Trokar nur ein Trokardorn sein, der in diesem Fall insbesondere ein optischer Trokar ist. Der optische Trokar weist eine durchsichtige distale Spitze auf sowie einen Innenkanal, in den eine Optik eingesetzt bzw. eingeführt wird.

Vorzugsweise weist der Trokar eine Trokarhülse auf, die mittels eines koaxialen Trokardorns durch eine Hautinzision z. B. in die Bauchdecke eingesetzt wird. Der Trokardorn kann dabei als optischer Trokar ausgebildet sein. Falls eine den Zugangskanal bildende Trokarhülse eingesetzt wird, wird in diese vorzugsweise eine Optik eingesetzt, die zur Ausleuchtung des Operationsfeldes dient und zur Bildgebung und Beobachtung mit einer einfachen Kamera oder einer 3D-Kamera ausgestattet ist. Der wesentliche Gedanke der Erfindung besteht darin, dass an dem Trokar wenigstens ein flexibel biegsamer Instrumentenkanal angeordnet ist, der zumindest in dem bei eingesetztem Trokar intrakorporal verbleibenden Bereich an der Außenseite des Trokars verläuft. Der Instrumentenkanal kann vorzugsweise eine schlauchförmige Hülle, z.B. ein Kunststoffschlauch sein. Der Instrumentenkanal verläuft in Längsrichtung des Trokars. Das distale Ende des Instrumentenkanals ist an dem distalen Ende des Trokars fixiert. In einem proximalen Längenabschnitt ist der Instrumentenkanal axial verschiebbar an dem Trokar geführt. Proximal versetzt gegen das distale Ende weist der Instrumentenkanal eine seitliche Austrittsöffnung auf. Ein semiflexibles Miniaturinstrument wird am proximalen Ende in den

Instrumentenkanal eingeführt und tritt mit seinem distalen Arbeitselement durch die Austrittsöffnung des Instrumentenkanals aus. Ist der Instrumentenkanal in der Führung an dem Trokar in proximaler Richtung geschoben, so liegt er in dem Bereich zwischen seiner distalen Fixierung und der Führung an der Außenwandung des Trokars an. Wird der Instrumentenkanal in der Führung in distaler Richtung geschoben, so biegt sich der Führungskanal auf Grund seiner distalen Fixierung zwischen dieser Fixierung und der Führung an dem Trokar bogenförmig nach außen. Die Austrittsöffnung des Instrumentenkanals befindet sich dabei in dem Bereich dieser Biegung. Das semiflexible Instrument tritt dadurch tangential zu der Biegung des Instrumentenkanals aus der Austrittsöffnung aus. Je weiter der Instrumentenkanal in distaler Richtung geschoben wird um so stärker wird die Krümmung der Biegung des Instrumentenkanals im distalen Bereich. Dadurch verlagert sich die Austrittsöffnung zunehmend in einen Bereich des Instrumentenkanals, der distal über das distale Ende des Trokars hinaus gebogen ist und unter einem zunehmenden Winkel gegen die geometrische Mittelachse des Trokars angestellt ist. Der Anstellwinkel des tangential aus dem Instrumentenkanal austretenden distalen Endes des Instruments gegenüber der Achse des Trokars kann dadurch zwischen 0° und mehr als 90° variiert werden. 0° bedeutet dabei parallel zur Achse, 90° bedeutet senkrecht zur Achse und mehr als 90° bedeutet dabei, dass das distale Ende des Instruments in proximaler Richtung gegen das Ende des Trokars gerichtet ist.

In einer bevorzugten Ausführung sind an dem Trokar zwei diametral zueinander angeordnete Instrumentenkanäle vorgesehen. Die durch diese Instrumentenkanäle zugeführten Instrumente sind somit mit ihren Arbeitselementen gegeneinander gerichtet angeordnet. Der Anstellwinkel der Instrumente gegenüber der Achse des Trokars und gegenüber dem jeweils anderen Instrument kann durch axiales Verschieben des jeweiligen Instrumentenkanals unabhängig variiert werden. Durch unterschiedlich weites Ausfahren des distalen Endes der jeweiligen Instrumente aus dem zugehörigen Instrumentenkanal kann dabei außerdem der radiale Abstand des jeweiligen Arbeitselements in Bezug auf die Trokarachse und in Bezug auf das jeweils andere Instrument eingestellt werden. Durch Drehen des extrakorporalen Betätigungsgriffes können die Instrumente um ihre Achse gedreht werden, sodass auch deren distale Arbeitselemente um die Instrumentenachse rotierbar verstellt werden können. Schließlich kann der Trokar selbst gekippt und um seine Achse gedreht werden, sodass die Ausrichtung der Instrumentenkanäle in Bezug auf den Operationssitus frei gewählt werden kann. Durch diese unterschiedlichen Einstellmöglichkeiten ergeben sich über den Stand der Technik hinaus weitere Freiheitsgrade für die Handhabung der Instrumente.

Das erfindungsgemäße Instrumentensystem eignet sich auch für eine robotergesteuerte Operation. Hierzu werden der Trokar, die proximalen Enden der Instrumentenkanäle und die Betätigung der Instrumente an steuerbare Achsen eines Roboters angeschlossen. Die große Zahl der Freiheitsgrade ermöglicht eine variable und präzise Manipulation der Arbeitselemente der Instrumente mit einer relativ einfachen Steuerung.

Sofern der Trokar nur einen Trokardorn aufweist, der als optischer Trokar ausgebildet ist, sind die Arbeitskanäle an diesem Trokardorn angebracht. Durch den optischen Trokar kann der operative Eingriff mittels der Instrumente beobachtet und unter Sicht durchgeführt werden. Bevorzugt ist eine Ausführung, bei welcher der Trokar aus einem Trokardorn und einer Trokarhülse besteht. In diesem Fall sind die Instrumentenkanäle an der Trokarhülse angeordnet. Die Trokarhülse mit den Instrumentenkanälen wird mittels des Trokardorns eingesetzt. Anschließend wird der Trokardorn entfernt und eine Optik in die Trokarhülse eingesetzt, die das Ausleuchten und Beobachten des Operationsfeldes bewirkt, sodass der Eingriff mittels der Instrumente unter Sicht erfolgen kann.

Im Folgenden wird die Erfindung anhand eines in der Zeichnung dargestellten Ausführungsbeispieles näher erläutert. Es zeigen:
- Fig. 1: die Verwendung eines Instrumentensystems gemäß der Erfindung bei einer minimalinvasiven Operation und
- Figuren 2 bis 4: das distale Ende des Instrumentensystems in unterschiedlichen Positionen der Instrumentenkanäle.

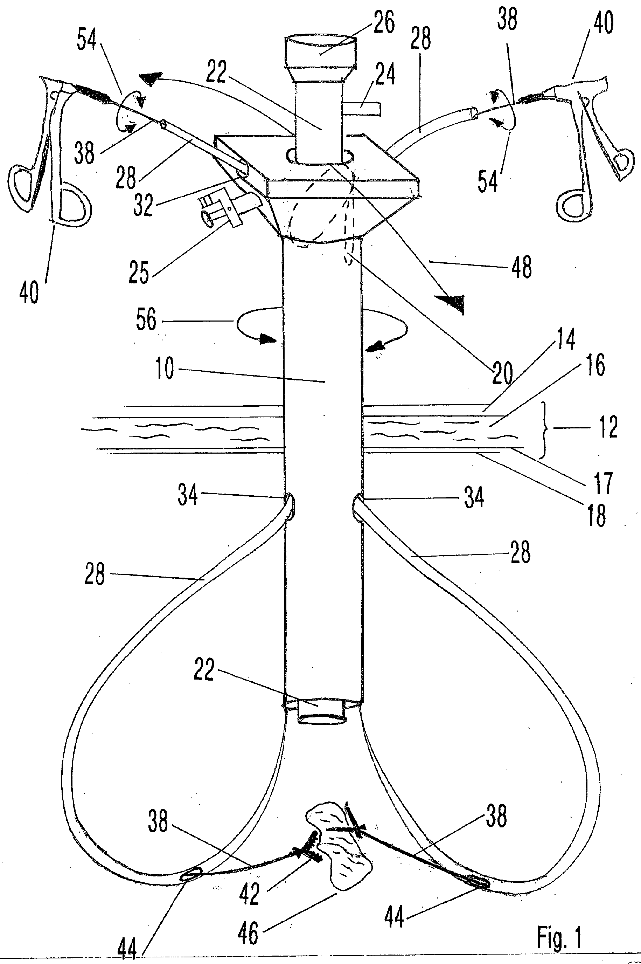

In Figur 1 ist das Instrumentensystem gemäß der Erfindung bei einem laparoskopischen Eingriff dargestellt.

Ein Trokar besteht im dargestellten Ausführungsbeispiel aus einer Trokarhülse 10 und einem in der Zeichnung nicht dargestellten massiven oder optischen Trokardorn. Der Trokar wird in die Bauchdecke 12 des Patienten eingesetzt, wobei die distale Spitze des Trokardorns durch eine Inzision der Haut 14 eindringt und das Fettgewebe 16, die Faszie 17 und das Peritoneum 18 durchdringt. Sobald die distale Spitze des Trokars durch das Peritoneum 18 in den Bauchraum gelangt ist, wird in den meisten Fällen der Bauchraum insuffliert. Anschließend wird der Trokardorn aus der Trokarhülse 10 herausgezogen, die als Zugang in das intrakorporale Operationsfeld in der Bauchdecke 12 verbleibt. Ein Ventil 20 verschließt die Trokarhülse 10, damit das Insufflationsgas nicht entweicht. Insoweit entspricht das Instrumentensystem dem Stand der Technik.

Bei der Single-Port-Operationstechnik wird nur ein einziger solcher Zugang erzeugt und sowohl die Optik, die zum Ausleuchten und zum Beobachten des Operationsfeldes dient, als auch die für den operativen Eingriff eingesetzten Instrumente werden durch diesen einzigen Zugang eingeführt.

Durch das Innenlumen der rohrförmigen Trokarhülse 10 wird eine Optik 22 eingeführt, die distal am distalen Ende der Trokarhülse 10 endet. Die Optik 22 weist beispielsweise einen Lichtleiter 24 auf, der Licht einer extrakorporalen Lichtquelle zum distalen Ende der Optik leitet, um das Operationsfeld auszuleuchten. Weiter weist die Optik ein Abbildungssystem auf, durch welches das Operationsfeld beobachtet werden kann. Das Abbildungssystem kann beispielsweise eine Kamera 26 umfassen, die das Bild des Operationsfelds auf einen Monitor überträgt. Die Abbildung kann dabei zweidimensional oder auch dreidimensional sein. Solche Optiken 22 sind ebenfalls an sich bekannter Stand der Technik.

Erfindungsgemäß ist an dem Trokar wenigstens ein Instrumentenkanal 28 angeordnet. In dem dargestellten Ausführungsbeispiel sind zwei Instrumentenkanäle 28 vorgesehen, die in dem dargestellten Ausführungsbeispiel an der Trokarhülse 10 diametral zueinander angeordnet sind. Die Instrumentenkanäle 28 sind flexibel biegsam und in ihrer Längsrichtung stabil. Auf Grund dieser Eigenschaften können die Instrumentenkanäle 28 in ihrer Längsrichtung wirkende Druck- und Zugkräfte aufnehmen. Bei Einwirken einer axialen Druckkraft biegen sich die Instrumentenkanäle 28 und wölben sich seitlich aus. Vorzugsweise sind die Instrumentenkanäle 28 als schlauchförmige Hülle ausgebildet, wie sie beispielsweise als Hülle bei Bowdenzügen verwendet werden. Die Hülle kann vorzugsweise aus Kunststoff gebildet sein. Zweckmäßig können der Instrumentenkanal 28 und gegebenenfalls auch die Trokarhülse 10 aus einem durchsichtigen Material bestehen, so dass ein in dem Instrumentenkanal 28 geführtes Instrument 38 von außen sichtbar ist und seine Bewegung verfolgt werden kann. Die Hülle kann auch mit vorzugsweise axial verlaufenden Verstärkungen bzw. Versteifungen ausgebildet sein, die die Biegung des Instrumentenkanals 28 formgebend stabilisieren, sodass eine Biegung vorzugsweise in der Krümmungsebene stattfindet, während ein Ausweichen aus dieser Krümmungsebene erschwert ist.

Die Instrumentenkanäle 28 sind mit ihrem distalen Ende 30 am distalen Ende der Trokarhülse 10 fixiert. Hierzu kann beispielsweise das distale Ende 30 des Instrumentenkanals 28 haarnadelförmig umgebogen und axial mit dem distalen Ende der Trokarhülse 10 vergossen oder verklebt sein. Es ist auch möglich, den Befestigungspunkt des distalen Endes 30 des Instrumentenkanals 28 auf dem distalen Ende der Trokarhülse 10 mittels geeigneter Stellmittel axial verstellbar auszubilden. Der Instrumentenkanal 28 verläuft von seinem fixierten distalen Ende 30 frei in proximaler Richtung an der Außenseite der Trokarhülse 10 entlang. Proximal ist der Instrumentenkanal 28 mit einem mittleren Längenabschnitt axial verschiebbar an dem Trokar geführt. Die Führung kann in unterschiedlicher Weise ausgebildet sein, wobei wesentlich ist, dass der Instrumentenkanal 28 in Bezug auf den Trokar bzw. die Trokarhülse 10 axial verschiebbar ist, jedoch in radialer Richtung an dem Trokar bzw. der Trokarhülse 10 gehalten wird. Im dargestellten Ausführungsbeispiel ist die Führung an dem Trokar in der Weise realisiert, dass der Instrumentenkanal 28 proximal durch eine Eingangsöffnung 32 in die Trokarhülse 10 eintritt und im Inneren der Trokarhülse 10 zu einer distal versetzt angeordneten Ausgangsöffnung 34 verläuft, durch welche der Instrumentenkanal 28 wieder an die Außenseite der Trokarhülse 10 austritt. Dadurch ist der Instrumentenkanal 28 durch sein fixiertes distales Ende 30 einerseits und durch die Ausgangöffnung 34 andererseits an der Trokarhülse 10 definiert festgelegt. In dem Längenbereich 36 zwischen dem fixierten distalen Ende 30 und der durch die Ausgangsöffnung 34 definierten Führungsstelle ist der Instrumentenkanal 28 flexibel von der Außenwand der Trokarhülse 10 weg und nach vorn über das distale Ende der Trokarhülse 10 hinaus ausbiegbar. Die Führung des Instrumentenkanals 28 an dem Trokar bzw. im dargestellten Ausführungsbeispiel an der Trokarhülse 10 ist so ausgebildet, dass sich die Eingangsöffnung 32 extrakorporal und die Ausgangsöffnung 34 intrakorporal befinden, wenn der Trokar in die Bauchdecke 12 eingesetzt ist, wie dies in Figur 1 dargestellt ist. Für eine Insufflation der Bauchhöhle über einen Insufflationsanschluss 25 ist der Instrumentenkanal 28 vorzugsweise in der Eingangsöffnung 32 z. B. durch eine Lippendichtung abgedichtet geführt.

In das proximale Ende der Instrumentenkanäle 28 kann jeweils ein Instrument 38 für die minimalinvasive Operation eingeführt werden. Solche Instrumente 38 sind in der minimalinvasiven Chirurgie an sich bekannt und werden häufig als Miniaturinstrumente bezeichnet. Die Instrumente 38 zeichnen sich dadurch aus, dass sie einen langen semiflexiblen Schaft aufweisen, in welchem ein Betätigungsdraht geführt ist. Unter "semiflexibel" ist dabei ein Schaft zu verstehen, der biegsam ist, jedoch eine ausreichende Längssteifigkeit aufweist. Am proximalen Ende des Instruments ist ein Betätigungsgriff 40 angeordnet, z. B. ein Scherengriff. Am distalen Ende des Instruments 38 ist jeweils ein Arbeitselement angeordnet, welches mittels des Betätigungsgriffs 40 betätigbar ist. Solche Instrumente 38 sind in unterschiedlichsten Ausführungen für die jeweiligen Verwendungszwecke bekannt. Die Instrumente 38 können beispielsweise als Schere, als Klemme, als Fasszange, als Koagulator, als Nadelhalter, als Clips-Applikator usw. ausgebildet sein. Diese Instrumente 38 weisen einen geringen Durchmesser des semiflexiblen Schaftes und des Arbeitselements 42 von wenigen Millimetern, z. B. von ca. 2 mm auf. Die Instrumente 38 sind für eine Insufflation vorzugsweise im proximalen Ende des Instrumentenkanals 28 abgedichtet geführt.

Das Instrument 38 wird in das proximal aus der Eingangsöffnung 32 herausragende proximale Ende des Instrumentenkanals 28 eingeführt und durch den Instrumentenkanal 28 vorgeschoben. Der Instrumentenkanal 28 weist in seinem Mantel mindestens eine seitliche Austrittsöffnung 44 auf, die sich von dem fixierten distalen Ende 30 des Instrumentenkanals 28 in proximaler Richtung versetzt in dem sich ausbiegenden Längenbereich 36 befindet. Distal von der Austrittsöffnung 44 ist das Innenlumen des Instrumentenkanals 28 verschlossen, so dass der Instrumentenkanal 28 für das Einschieben des Instrumentes 38 in die Austrittsöffnung 44 mündet. Wird das Instrument 38 in dem Instrumentenkanal 28 vorgeschoben, so wird das distale Ende des Instruments 38 mit dem Arbeitselement 42 durch die Austrittsöffnung 44 distal herausgeschoben. Auf Grund der Semiflexibilität des Schaftes des Instruments 38 tritt dabei das distale Ende des Schaftes tangential zu dem Bogen des seitlich ausgewölbten Längenbereichs 36 aus. Mit Hilfe des extrakorporal verbleibenden Betätigungsgriffes 40 kann das Instrument 38 durch den Operateur gezielt in dem Instrumentenkanal 28 in Längsrichtung verschoben werden, sodass das distale Ende mit dem Arbeitselement 42 unterschiedlich weit distal aus der Austrittsöffnung 44 austritt. Weiter kann das Instrument 38 mittels des Betätigungsgriffs 40 in dem Instrumentenkanal 28 um seine Längsachse gedreht werden, sodass das Arbeitselement 42 um die Achse des Instrumentenschaftes rotiert werden kann. Während der Operation kann der Instrumentenkanal 28 an der proximalen Eingangsöffnung 32 durch geeignete Mittel in Bezug auf axiale und rotatorische Bewegungen arretiert werden.

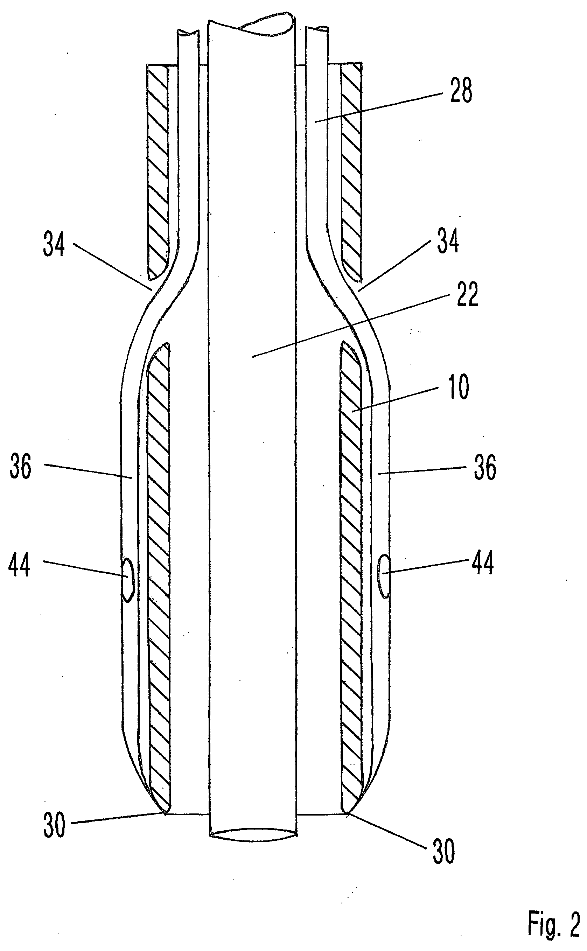

Ein minimalinvasiver chirurgischer Eingriff in der Single-Port-Technik mit dem erfindungsgemäßen Instrumentensystem verläuft in folgenden Schritten:
Nach einer Inzision in die Haut 14 wird der Trokar in die Bauchdecke 12 eingesetzt. Hierzu wird die Bauchdecke 12 mittels eines in die Trokarhülse 10 eingesetzten Trokardorns perforiert. Die Instrumentenkanäle 28 sind beim Einsetzen des Trokars mit der Trokarhülse 10 in proximaler Richtung gezogen, sodass die Instrumentenkanäle 28 mit ihrem ausbiegbaren Längenbereich 36 im Wesentlichen achsparallel an der Außenseite der Trokarhülse 10 anliegen, wie dies in Figur 2 gezeigt ist. Dadurch behindern die Instrumentenkanäle 28 das Eindringen des Trokars durch die Bauchdecke 12 nicht.

Sobald der Trokar in die Bauchdecke 12 eingesetzt ist wird die Bauchdecke 12 angehoben, z. B. durch Insufflation mittels des Insufflationsanschlusses 25, um einen ausreichenden Freiraum für die Operation zu schaffen. Dabei befinden sich die Ausgangsöffnungen 34 der Führung der Instrumentenkanäle 28 intrakorporal im Bauchraum, während sich die Eingangsöffnungen 32 extrakorporal befinden, wie dies Figur 1 zeigt. Der für das Penetrieren der Bauchdecke 12 und das Dilatieren des Einstichloches benötigte Trokardorn wird dann aus der Trokarhülse 10 herausgezogen und gegen eine in die Trokarhülse 10 eingesetzte Optik 22 ausgetauscht, die gegebenenfalls axial einstellbar und fixierbar ist.

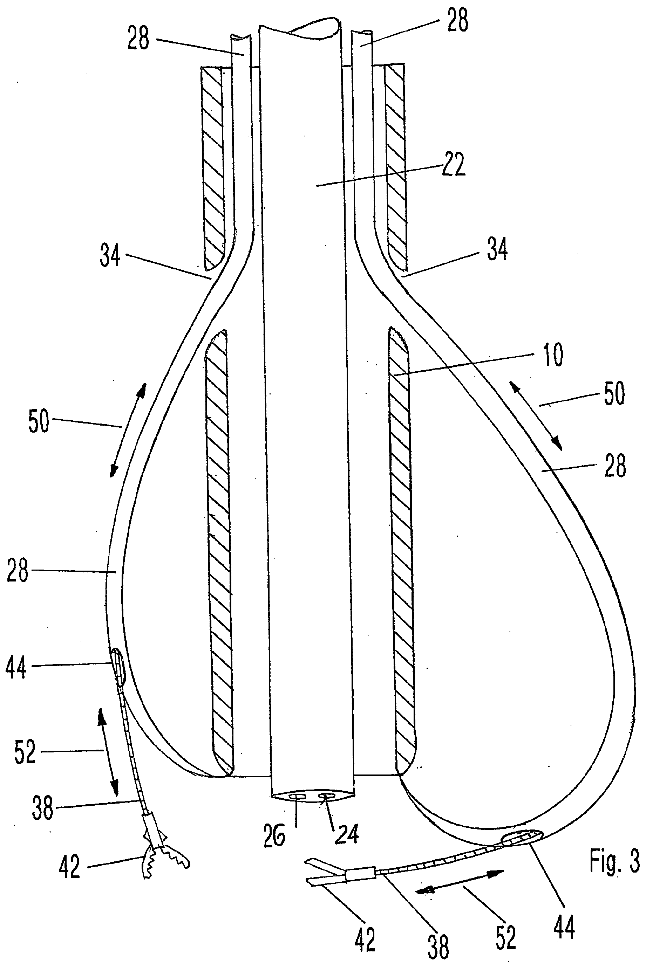

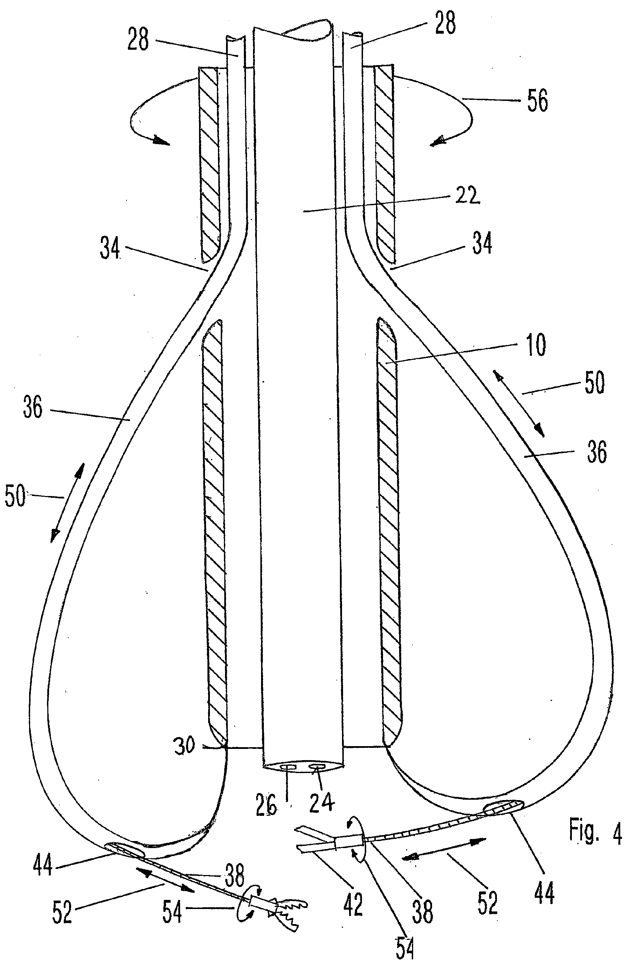

Die Instrumentenkanäle 28 werden an ihrem proximal freiliegenden Ende erfasst und durch die von der Eingangsöffnung 32 und der Ausgangsöffnung 34 gebildete Führung axial in distaler Richtung vorgeschoben. Dabei wölbt sich der Längenbereich 36 der Instrumentenkanäle 28 zunehmend seitlich und nach vorn über das distale Ende der Trokarhülse 10 hinaus aus, wie dies in Figur 3 für den linken und den rechten Instrumentenkanal 28 dargestellt ist. Die für den Eingriff benötigten Instrumente 38 werden in die Instrumentenkanäle 28 eingeführt. Das distale Ende der Instrumente 38 mit den jeweiligen Arbeitselementen 42 tritt dabei tangential zur Krümmung des ausgebogenen Instrumentenkanals 28 durch die jeweilige Austrittsöffnung 44 aus. Wie Figur 3 zeigt, wandert beim zunehmenden distalen Verschieben des Instrumentenkanals 28 die Austrittsöffnung 44 zunehmend in den Bereich des Instrumentenkanals 28 der stärker gegen die Trokarachse abgebogen ist. Der Anstellwinkel zwischen der Achse des tangential aus der Austrittsöffnung 44 austretenden distalen Endes des Instruments 38 gegenüber der Mittelachse des Trokars ändert sich dadurch zunehmend von etwa 0° (Figur 3 links) über etwa 90° (Figur 3 rechts und Figur 4 links) bis beispielsweise etwa 120° (Figur 4 rechts). Ein Winkel von 0° bedeutet dabei, dass das distale Ende des Instruments 38 etwa parallel zur Achse des Trokars austritt, ein Winkel von 90° bedeutet, dass das distale Ende des Instruments 38 im Wesentlichen senkrecht gegen die Mittelachse des Trokars austritt, während ein Winkel von 120° bedeutet, dass das distale Ende des Instruments 38 rückwärts gegen das distale Ende der Trokarhülse 10 gerichtet austritt.

Sind die Instrumentenkanäle 28 soweit ausgebogen, dass die distalen Enden der Instrumente 38 gegen die Mittelachse der Trokarhülse 10 und somit gegeneinander gerichtet sind, können mittels der Arbeitselemente 42 die erforderlichen chirurgischen Schritte an dem zu behandelnden Körpergewebe 46 ausgeführt werden, wie dies in Figur 1 gezeigt ist. Das Gewebe 46 und die Arbeitselemente 42 befinden sich dabei distal vor dem distalen Ende der Optik 22, sodass das Operationsfeld gut ausgeleuchtet ist und der Eingriff unter optimaler Sicht durchgeführt werden kann.

Ist der Eingriff beendet, so werden die Instrumente 38 aus den Instrumentenkanälen 28 herausgezogen. Die Instrumentenkanäle 28 werden wieder in proximaler Richtung zurückgezogen, sodass sie sich außen an die Trokarhülse 10 anlegen, wie dies in Figur 2 gezeigt ist. Die Trokarhülse kann nun unbehindert aus der Einstichöffnung der Bauchdecke 12 herausgezogen werden.

Das erfindungsgemäße Instrumentensystem ermöglicht eine außerordentlich große Zahl von Freiheitsgraden für den Einsatz der Instrumente 38, sodass eine Single-Port-Operation in besonders vielseitiger und zuverlässiger Weise durchgeführt werden kann. Der Trokar mit der Trokarhülse 10 kann in seinem Einstichwinkel in Bezug auf die Bauchdecke 12 gekippt werden, wie in Figur 1 durch den Pfeil 48 gezeigt ist. Dadurch kann die Trokarhülse 10 auf das intrakorporale Operationsfeld ausgerichtet werden. Die Instrumentenkanäle 28 können in der Führung 32, 34 axial verschoben werden, wie durch die Pfeile 50 angedeutet ist. Dadurch kann der Austrittswinkel der distalen Enden der Instrumente 38 variiert werden. Die Instrumente 38 können in den Instrumentenkanälen 28 unterschiedlich weit axial verschoben werden, wie durch die Pfeile 52 angedeutet ist. Dadurch können die Arbeitselemente 42 gezielt unterschiedlich weit an die Mittelachse des Instrumentensystems herangeführt werden. Die Instrumente 38 können in den Instrumentenkanälen 28 gedreht werden, sodass die Arbeitselemente 42 um die Instrumentenachse rotiert werden können, wie durch die Pfeile 54 gezeigt ist. Dadurch kann der Eingriffswinkel der Arbeitselemente 42 variiert werden. Schließlich kann die Trokarhülse 10 um ihre Mittelachse rotiert werden, wie durch den Pfeil 56 angedeutet ist. Dadurch können die Instrumentenkanäle 28 und damit die Eingriffsrichtung der Arbeitselemente 42 um die Mittelachse und damit um den Eingriffsort 46 gedreht werden.

In dem dargestellten Ausführungsbeispiel liegen die Instrumentenkanäle 28 in der zurückgezogenen Position gemäß Figur 2 außen am Mantel der Trokarhülse 10 an. In einer anderen Ausführung können in der äußeren Mantelfläche der Trokarhülse 10 Längsnuten vorgesehen sein, die die Instrumentenkanäle 28 in der zurückgezogenen Stellung aufnehmen. Dadurch kann der Einfluss der Instrumentenkanäle 28 beim Einstechen und beim Herausziehen des Trokars zusätzlich reduziert werden.

Weiter können im Bereich der Eingangsöffnung 32 Arretierungsmittel vorgesehen sein, die eine Blockierung der Axialverschiebung der Instrumentenkanäle 28 während des Eingriffs ermöglichen.

In dem dargestellten Ausführungsbeispiel weist der Trokar einen Trokardorn und eine als Zugangskanal verbleibende Trokarhülse 10 auf, in welche eine Optik 22 eingesetzt wird. Es ist auch möglich, den Trokar als optischen Trokar ohne eine Trokarhülse auszubilden. Dabei wird eine in den zur Perforation dienenden Trokardorn eingesetzte Optik auch zur Beobachtung des Operationsfeldes während des Eingriffs benutzt. In dieser Ausführung sind die Instrumentenkanäle außen an dem Trokardorn angeordnet, distal an diesem fixiert und mit ihrem mittleren Längenabschnitt an diesem Trokardorn geführt.

### Bezugszeichenliste

- 10: Trokarhülse
- 12: Bauchdecke
- 14: Haut
- 16: Fettgewebe
- 17: Faszie
- 18: Peritoneum
- 2.0: Ventil
- 22: Optik
- 24: Lichtleiter
- 25: Insufflationsanschluss
- 26: Kamera
- 28: Instrumentenkanal
- 30: distales Ende von 28
- 32: Eingangsöffnung
- 34: Ausgangsöffnung
- 36: Längenbereich
- 38: Instrument
- 40: Betätigungsgriff
- 42: Arbeitselement
- 44: Austrittsöffnung
- 46: Gewebe
- 48: Kippen des Trokars
- 50: Austrittswinkel
- 52: Austrittslänge
- 54: Rotation des Instruments
- 56: Rotation des Trokars

## Patentansprüche

1. Instrumentensystem für die minimalinvasive Chirurgie in der Single-Port-Technik, mit einem Trokar und mit wenigstens einem Instrumentenkanal (28) der an dem Trokar von proximal nach distal verläuft und ein bei in den Körper eines Patienten eingesetztem Trokar extrakorporal verbleibendes proximales Ende und ein sich intrakorporal befindendes distales Ende aufweist, wobei durch den Instrumentenkanal (28) ein semiflexibles Instrument (38) in der Weise einführbar ist, dass dessen Betätigungsgriff (40) extrakorporal verbleibt und dessen distales Arbeitselement (42) distal aus dem Instrumentenkanal (28) austritt,
**dadurch gekennzeichnet, dass** der Instrumentenkanal (28) flexibel biegsam und in seiner Längsrichtung stabil ist, dass das distale Ende (30) des Instrumentenkanals an dem Trokar fixiert ist, dass der Instrumentenkanal (28) proximal mit einem mittleren Abschnitt in einer Führung an dem Trokar axial verschiebbar ist, wodurch der Instrumentenkanal (28) in seinem Längenbereich (36) zwischen dem fixierten distalen Ende (30) und der Führung an dem Trokar außen an dem Trokar angeordnet ist und seitlich von dem Trokar weg und nach vorn über das distale Ende des Trokars hinaus ausgebogen wird, wenn der Instrumentenkanal (28) in der Führung nach distal geschoben wird, und dass der Instrumentenkanal (28) eine Austrittsöffnung (44) für das distale Ende des Instruments (38) aufweist, die sich von dem fixierten distalen Ende (30) in proximaler Richtung versetzt in dem sich ausbiegenden Längenbereichen (36) befindet.

2. Instrumentensystem nach Anspruch 1,
**dadurch gekennzeichnet, dass** der Instrumentenkanal (28) durch eine schlauchförmige Hülle gebildet ist.

3. Instrumentensystem nach Anspruch 1 oder 2,
**dadurch gekennzeichnet, dass** der Instrumentenkanal (28) in seinem nicht ausgebogenen Zustand an der äußeren Mantelfläche des Trokars achsparallel anliegt oder in einer äußeren Längsnut des Trokars aufgenommen ist.

4. Instrumentensystem nach einem der vorhergehenden Ansprüche,
**dadurch gekennzeichnet, dass** der Instrumentenkanal (28) in der Führung innerhalb des Trokars von einer extrakorporal verbleibenden Eingangsöffnung (32) zu einer intrakorporal liegenden Ausgangsöffnung (34) verläuft.

5. Instrumentensystem nach einem der vorhergehenden Ansprüche,
**dadurch gekennzeichnet, dass** zwei Instrumentenkanäle (28) diametral zueinander an dem Trokar angeordnet sind.

6. Instrumentensystem nach einem der vorhergehenden Ansprüche,
**dadurch gekennzeichnet, dass** der Trokar eine Trokarhülse (10) und einen in diese einsetzbaren Trokardorn aufweist, wobei der wenigstens eine Instrumentenkanal (28) an der Trokarhülse (10) angeordnet ist.

7. Instrumentensystem nach einem der vorhergehenden Ansprüche,
**dadurch gekennzeichnet, dass** der Trokar mit einer koaxial einsetzbaren Optik (22) ausgestattet ist, die gegebenenfalls axial verschiebbar und arretierbar ist.

8. Instrumentensystem nach den Ansprüchen 6 und 7,
**dadurch gekennzeichnet, dass** die Optik (22) nach dem Entfernen des Trokardorns in die Trokarhülse (10) einsetzbar ist.

9. Instrumentensystem nach Anspruch 7,
**dadurch gekennzeichnet, dass** der Trokar ein optischer Trokar mit einer durchsichtigen distalen Spitze ist, bei welchem eine Optik in den Trokardorn einsetzbar ist.

10. Instrumentensystem nach einem der vorhergehenden Ansprüche,
**dadurch gekennzeichnet, dass** der wenigstens eine Instrumentenkanal (28) an dem Trokar gegen axiale und rotatorische Bewegungen arretierbar ist.

11. Instrumentensystem nach einem der vorhergehenden Ansprüche,
**dadurch gekennzeichnet, dass** das Instrument (38) in dem Instrumentenkanal (28) abgedichtet geführt ist.

12. Instrumentensystem nach Anspruch 4,
**dadurch gekennzeichnet, dass** der Instrumentenkanal (28) in dem Trokar abgedichtet geführt ist.

13. Instrumentensystem nach einem der vorhergehenden Ansprüche,
**dadurch gekennzeichnet, dass** das distale Ende (30) des Instrumentenkanals (28) in axial verstellbarer Position an dem Trokar fixiert ist.

14. Instrumentensystem nach einem der vorhergehenden Ansprüche,
**dadurch gekennzeichnet, dass** der Instrumentenkanal (28) aus einem durchsichtigen Material besteht.

## Claims

1. Instrument system for minimally invasive surgery in single port technology, comprising a trocar and comprising at least one instrument channel (28) which runs from proximal to distal on the trocar and which has a proximal end that remains extracorporeal when the trocar is inserted into the body of a patient and a distal end that is located intracorporeally, wherein a semi-flexible instrument (38) can be inserted through the instrument channel (28) in such a way that the operating handle (40) of said semi-flexible instrument remains extracorporeal and the distal working element (42) thereof emerges distally from the instrument channel (28), **characterized in that** the instrument channel (28) is flexibly bendable and is stable in its longitudinal direction, **in that** the distal end (30) of the instrument channel is fixed on the trocar, **in that** the instrument channel (28) can be axially displaced proximally with a middle section in a guide on the trocar, as a result of which the instrument channel (28) is arranged, in its length region (36) between the fixed distal end (30) and the guide on the trocar, externally on the trocar and is bent laterally outwards away from the trocar and forwards beyond the distal end of the trocar when the instrument channel (28) is pushed in the distal direction in the guide, and **in that** the instrument channel (28) has an exit opening (44) for the distal end of the instrument (38), which exit opening is located in the outwardly bending length region (36) that is offset in the proximal direction from the fixed distal end (30).

2. Instrument system according to claim 1, **characterized in that** the instrument channel (28) is formed by a tubular sheath.

3. Instrument system according to claim 1 or 2, **characterized in that** the instrument channel (28), in its non-bent-out state, bears in an axis-parallel manner against the outer lateral surface of the trocar or is accommodated in an external longitudinal groove of the trocar.

4. Instrument system according to any of the preceding claims, **characterized in that** the instrument channel (28) runs in the guide within the trocar from an inlet opening (32) that remains extracorporeal to an outlet opening (34) that is located intracorporeally.

5. Instrument system according to any of the preceding claims, **characterized in that** two instrument channels (28) are arranged diametrically opposite one another on the trocar.

6. Instrument system according to any of the preceding claims, **characterized in that** the trocar has a trocar sleeve (10) and a trocar pin that can be inserted therein, wherein the at least one instrument channel (28) is arranged on the trocar sleeve (10).

7. Instrument system according to any of the preceding claims, **characterized in that** the trocar is equipped with an optical system (22) which can be inserted coaxially and which can optionally be axially displaced and locked.

8. Instrument system according to claims 6 and 7, **characterized in that** the optical system (22) can be inserted into the trocar sleeve (10) after removal of the trocar pin.

9. Instrument system according to claim 7, **characterized in that** the trocar is an optical trocar having a transparent distal tip, in which case an optical system can be inserted into the trocar pin.

10. Instrument system according to any of the preceding claims, **characterized in that** the at least one instrument channel (28) can be locked on the trocar against axial and rotational movements.

11. Instrument system according to any of the preceding claims, **characterized in that** the instrument (38) is guided in a sealed manner in the instrument channel (28).

12. Instrument system according to claim 4, **characterized in that** the instrument channel (28) is guided in a sealed manner in the trocar.

13. Instrument system according to any of the preceding claims, **characterized in that** the distal end (30) of the instrument channel (28) is fixed in an axially adjustable position on the trocar.

14. Instrument system according to any of the preceding claims, **characterized in that** the instrument channel (28) is made of a transparent material.

## Revendications

1. Système d'instruments destiné à la chirurgie mini-invasive selon la technique Single-Port comprenant un trocart et au moins un canal d'instruments (28) qui s'étend sur le trocart, de son extrémité proximale vers son extrémité distale, et, comporte, lorsque le trocart est introduit dans le corps d'un patient, une extrémité proximale restant extracorporelle et une extrémité distale située dans une position intracorporelle, dans lequel au travers du canal d'instruments (28) peut être introduit un instrument semi-flexible (38) de façon que sa poignée de commande (40) reste dans une position extracorporelle et que son élément de travail distal (42) sorte du canal d'instruments (28) à son extrémité distale,
**caractérisé en ce que**
le canal d'instruments (28) est souple et flexible et est stable dans sa direction longitudinale, l'extrémité distale (30) du canal d'instruments est fixée au trocart, le canal d'instruments (28) peut être déplacé par coulissement axial à sa partie proximale par un segment médian dans un guidage du trocart, de sorte que le canal d'instruments (28) soit positionné extérieurement sur le trocart, dans sa zone longitudinale (36) située entre son extrémité distale fixe (30) et le guidage du trocart, et puisse être courbé latéralement en s'écartant du trocart et vers l'avant au-delà de l'extrémité distale du trocart, lorsque le canal d'instruments (28) coulisse dans le canal vers l'extrémité distale, et le canal d'instruments (28) comporte une ouverture de sortie (44) pour l'extrémité distale de l'instrument (38) qui est située dans la zone longitudinale qui se courbe (36) en étant décalée par rapport à l'extrémité distale fixe (30) dans la direction proximale.

2. Système d'instruments conforme à la revendication 1,
**caractérisé en ce que**
le canal d'instruments (28) est fermé par une gaine en forme de tuyau.

3. Système d'instruments conforme à la revendication 1 ou 2,
**caractérisé en ce que**
dans son état non recourbé, le canal d'instruments (28) s'applique sur la surface enveloppe externe du trocart, parallèlement à son axe ou est logé dans une rainure longitudinale externe du trocart.

4. Système d'instruments conforme à l'une des revendications précédents,
**caractérisé en ce que**
le canal d'instruments (28) s'étend dans le guidage situé à la partie interne du trocart d'une ouverture d'entrée (32) restant dans une position extracorporelle à une ouverture de sortie (34) située dans une position intracorporelle.

5. Système d'instruments conforme à l'une des revendications précédentes,
**caractérisé en ce que**
deux canaux d'instruments (28) diamétralement opposés sont situés sur le trocart.

6. Système d'instruments conforme à l'une des revendications précédentes,
**caractérisé en ce que**
le trocart comporte une gaine de trocart (10) et une broche de trocart pouvant être introduite dans celle-ci, le canal d'instruments (28) étant situé sur la gaine de trocart (10).

7. Système d'instruments conforme à l'une des revendications précédentes,
**caractérisé en ce que**
le trocart est équipé d'une optique (22) pouvant être introduite co-axialement qui, le cas échéant peut coulisser axialement et être bloquée.

8. Système d'instruments conforme aux revendications 6 et 7,
**caractérisé en ce que**
l'optique (22) peut être mise en place dans la gaine de trocart (10) après extraction de la broche de trocart.

9. système d'instruments conforme à la revendication 7,
**caractérisé en ce que**
le trocart est un trocart optique ayant une pointe distale transparente, une optique pouvant être introduite dans la broche de trocart.

10. Système d'instruments conforme à l'une des revendications précédentes,
**caractérisé en ce que**
le canal d'instruments (28) situé sur le trocart peut être bloqué vis-à-vis de mouvements axiaux et de mouvements de rotation.

11. Système d'instruments conforme à l'une des revendications précédentes,
**caractérisé en ce que**
l'instrument (38) est guidé hermétiquement dans le canal d'instruments (28).

12. Système d'instruments conforme à la revendication 4,
**caractérisé en ce que**
le canal d'instruments (28) est guidé hermétiquement dans le trocart.

13. Système d'instruments conforme à l'une des revendications précédentes,
**caractérisé en ce que**
l'extrémité distale (30) du canal d'instruments (28) est fixé sur le trocart dans une position réglable axialement.

14. Système d'instruments conforme à l'une des revendications précédentes,
**caractérisé en ce que**
le canal d'instruments (28) est réalisé en un matériau transparent.
